## Europäisches Patentamt
## European Patent Office
## Office européen des brevets

(19) 

(11) Numéro de publication: **0 191 747**
**B1**

(12) **FASCICULE DE BREVET EUROPÉEN**

(45) Date de publication du fascicule du brevet:
18.05.88

(51) Int. Cl.⁴: **A 61 F 5/47**

(21) Numéro de dépôt: **86870012.1**

(22) Date de dépôt: **29.01.86**

(54) **Dispositif anticonceptionnel intra-utérin nouveau et perfectionné et son dispositif d'insertion et de fixation de la matrice.**

(30) Priorité: **05.02.85 BE 214449**

(43) Date de publication de la demande:
**20.06.86 Bulletin 86/34**

(45) Mention de la délivrance du brevet:
**18.05.88 Bulletin 88/20**

(84) Etats contractants désignés:
**BE CH DE FR GB IT LI NL SE**

(56) Documents cité:
**EP-A-0 100 924**
**EP-A-0 147 274**
**BE-A-899 286**
**BE-A-901 652**
**DE-A-2 207 939**
**DE-A-3 209 290**
**FR-A-2 165 714**
**FR-A-2 253 537**
**US-A-1 896 071**
**US-A-3 820 535**
**US-A-3 954 103**
**US-A-4 005 707**
**US-A-4 294 260**

(73) Titulaire: **Wildemeersch, Dirk, Vossenhul 8, B-8300 Knokke- Heist (BE)**

(72) Inventeur: **Wildemeersch, Dirk, Vossenhul 8, B-8300 Knokke- Heist (BE)**

(74) Mandataire: **Prignot, Jean, OFFICE HANSSENS SPRL Square Marie- Louise, 40 - bte 19, B-1040 Bruxelles (BE)**

## Description

La présente invention a pour objet un dispositif anticonceptionnel intra-utérin nouveau et perfectionné, remarquablement bien toléré par la matrice, et qui présente également une très grande efficacité par l'importance de la surface utile de cuivre mise en oeuvre. En outre, la conception du dispositif anticonceptionnel intra-utérin de l'invention permet son placement aisé dans la matrice, même de femmes qui n'ont pas encore été gravides, à l'aide d'un dispositif de très faible encombrement.

La plupart des dispositifs anticonceptionnels intra-utérins connus sont constitués d'un support, de forme géométrique déterminée pour permettre sa rétention dans la matrice, sur lequel sont portés des enroulements de cuivre. La forme géométrique de ces supports, qui sont généralement en T ou en Y, est conçue pour que les dits supports s'adaptent à la forme interne, en triangle renversé, de la cavité utérine. Toutefois, la diversité en taille des utérus nécessite une adaptation du dispositif anticonceptionnel intra-utérin à la taille de l'utérus. En outre, sous l'effet des contractions de l'utérus, des translocations du dispositif anticonceptionnel intra- utérin demeurent constamment possibles. Enfin, il faut signaler que la rigidité relative de tels dispositifs pose très fréquemment des problèmes de tolérance, cette rigidité entraînant dans de nombreux cas des douleurs, des saignements de l'utérus, et pouvant même être responsables de dégâts importants à l'utérus (percement de la paroi).

Quant à l'efficacité de ces dispositifs, elle dépend de la surface de cuivre mise en oeuvre et de la position correcte du cuivre dans l'utérus. Dans de tels dispositifs, il est généralement admis qu'environ 60 % de la surface de cuivre présent sur le stérilet a un rôle effectif, les 40 % restants se retrouvant au contact du support, et ne jouant donc pas de rôle contraceptif. En outre, en cas de position incorrecte du dispositif anticonceptionnel intra-utérin, l'action du cuivre s'exerce de manière inégale sur les parois de la matrice.

Il a déjà été proposé, dans la demande de brevet EP-A-0 100 924, de réaliser le dispositif anticonceptionnel sous forme d'une simple chaînette de cuivre, fixée à la paroi du fond de la matrice. Un dispositif anticonceptionnel de ce type présente l'avantage de ne pas avoir de forme propre, et donc de s'adapter sans aucune contrainte aux mouvements de la matrice. Il est donc parfaitement toléré et, dans la mesure où il est fixé de manière bien centrée au fond de la matrice, exerce son action de manière symétrique sur les parois de cette dernière.

Le dispositif préconisé à la demande de brevet EP-A-0 100 924 précitéee présente cependant l'inconvénient de faire usage d'un dispositif compliqué de fixation à la paroi de la matrice, ne permettant pas un retrait aisé du dispositif anticonceptionnel intra-utérin, et nécessitant un dispositif de mise en place relativement encombrant. En outre, la surface de cuivre de la chaînette est peu importante. Enfin, au cours du temps, l'attaque du cuivre peut provoquer, par rupture d'un maillon, l'élimination d'une part importante, sinon de la totalité, du cuivre présent dans la matrice, suivant que le maillon qui cède se trouve plus ou moins proche du dispositif de fixation.

L'invention a pour but de remédier à ces divers inconvénients en prévoyant un dispositif anticonceptionnel intra-utérin de grande surface active, susceptible d'être introduit dans la matrice et fixé à la paroi de cette dernière à l'aide d'un dispositif de très faible section, permettant un passage aisé au-travers du col de la matrice, même de femmes qui n'ont pas encore été gravides.

Ces buts sont atteints suivant l'invention à l'aide d'un dispositif anticonceptionnel intra-utérin, comportant un dispositif de fixation à la paroi du fond de la matrice solidaire d'éléments en un matériau qui est actif dans la cavité de la matrice, réunis à la file en un assemblage non rigide, caractérisé en ce que les éléments en un matériau actif dans la cavité de la matrice sont des éléments creux, percés de part en part et disposés bout à bout, éventuellement espacés entre eux tout en étant rattachés l'un à l'autre, pour former un conduit longitudinal permettant le passage d'une aiguille, et dans lequel le dispositif de fixation à la paroi du fond de la matrice est un fil solidaire de l'assemblage d'éléments creux et pourvu d'un dispositif d'accrochage au tissu de la matrice destiné à être inséré à l'aide d'une aiguille.

Suivant une autre caractéristique de l'invention, les éléments creux sont enfilés sur le fil pourvu d'un dispositif d'accrochage au tissu de la matrice, et sont retenus au moins par un dispositif d'arrêt formé sur ce fil à l'opposé du dispositif d'accrochage au tissu de la matrice, le fil réalisant un moyen d'assemblage des éléments du dispositif anticonceptionnel intra-utérin au moins lorsque le dispositif d'accrochage est inséré dans le tissu de la matrice.

Suivant une caractéristique supplémentaire, le dispositif d'arrêt sur le fil est constitué par la solidarisation du fil à l'élément creux le plus éloigné du dispositif d'accrochage au tissu de la matrice.

Suivant encore une caractéristique supplémentaire de l'invention, un second dispositif d'arrêt est formé sur le fil, par solidarisation du fil à l'élément creux le plus proche du dispositif d'accrochage au tissu de la matrice, la distance entre les deux dispositifs d'arrêt assurant un assemblage lâche des éléments creux.

Suivant une autre caractéristique de l'invention, en vue du traitement de la matrice, les éléments creux, ou au moins certains d'entre eux, sont réalisés en une matière consommable libérant un agent de traitement dans la matrice.

Suivant encore une autre caractéristique de l'invention, les éléments creux sont assemblés l'un à l'autre par des maillons réalisant un assemblage non rigide, et l'un au moins des éléments creux ou des maillons est solidaire du fil pourvu du moyen d'accrochage à la matrice.

Suivant une autre caractéristique de l'invention, les éléments creux sont des éléments tubulaires en cuivre.

Suivant encore une autre caractéristique de l'invention, au-delà de son extrémité opposée au dispositif d'accrochage de la matrice, le dispositif anticonceptionnel intra-utérin se prolonge par un fil permettant d'assurer une traction sur le dit dispositif d'accrochage à la matrice.

Suivant une caractéristique complémentaire de l'invention, le fil permettant d'assurer une traction sur le dispositif d'accrochage au tissu de la matrice est le fil portant le dit dispositif d'accrochage.

Un autre objet de l'invention est de fournir un dispositif d'insertion et de fixation à la matrice muni d'un dispositif anticonceptionnel intra-utérin suivant l'invention, qui comprend une aiguille pour l'introduction de l'élément d'accrochage solidaire du fil dans le tissu de la matrice, un élément de protection de l'aiguille et de réception du dispositif anticonceptionnel intra-utérin, un élément d'actionnement de l'aiguille, mobile par rapport à l'élément de protection, dispositif d'insertion dans lequel l'aiguille traverse le conduit formé dans le dispositif anticonceptionnel intra-utérin pour venir en prise avec le dispositif d'accrochage au tissu de la matrice, et la section interne de l'élément de protection de l'aiguille et de réception du dispositif anticonceptionnel intra-utérin correspond substantiellement à la section transversale externe des éléments creux du dispositif anticonceptionnel intra-utérin.

Suivant une autre caractéristique de l'invention, le dispositif d'insertion comprend un moyen de blocage de l'extrémité du fil opposée à l'élément d'accrochage, et une butée limitant le recul de l'élément de protection sur l'aiguille.

Suivant une caractéristique complémentaire de l'invention, le moyen de blocage du fil est formé sur l'aiguille et est constitué d'une fente dans la butée.

Suivant encore une autre caractéristique de l'invention, le dispositif d'insertion présente des moyens de solidarisation libérables de l'élément de protection à l'aiguille.

L'invention sera mieux comprise en se reportant à la description en même temps qu'au dessin annexé qui représente, uniquement à titre d'exemple, divers modes de réalisation de l'invention, et dans lequel:

- la fig. 1 montre, en coupe, un mode de réalisation d'un dispositif anticonceptionnel intra-utérin suivant l'invention.

- la fig. 2 représente, également en coupe, un autre mode de réalisation d'un dispositif anticonceptionnel intra- utérin suivant l'invention,

- la fig. 3 est une vue en coupe d'un troisième mode de réalisation d'un dispositif anticonceptionnel intra-utérin suivant l'invention,

- la fig. 4 montre en coupe un dispositif d'insertion d'un dispositif anticonceptionnel intra-utérin dans la matrice,

- la fig. 5 est une vue partielle d'une variante de réalisation du dispositif de la fig. 4,

- la fig 6 est une vue éclatée, en perspective, d'un mode de réalisation préféré d'un dispositif d'insertion d'un dispositif anticonceptionnel intra-utérin dans la matrice,

- la fig 7 représente un autre mode de réalisation d'un dispositif d'insertion suivant l'invention, en position inséré dans la matrice,

- la fig 8 est une vue partielle d'un dispositif d'insertion inséré dans la matrice, après pénétration de l'aiguille dans la paroi de la matrice, et

- la fig 9 est une vue schématique en coupe de la matrice dans laquelle a été placé un dispositif anticonceptionnel intra-utérin suivant l'invention.

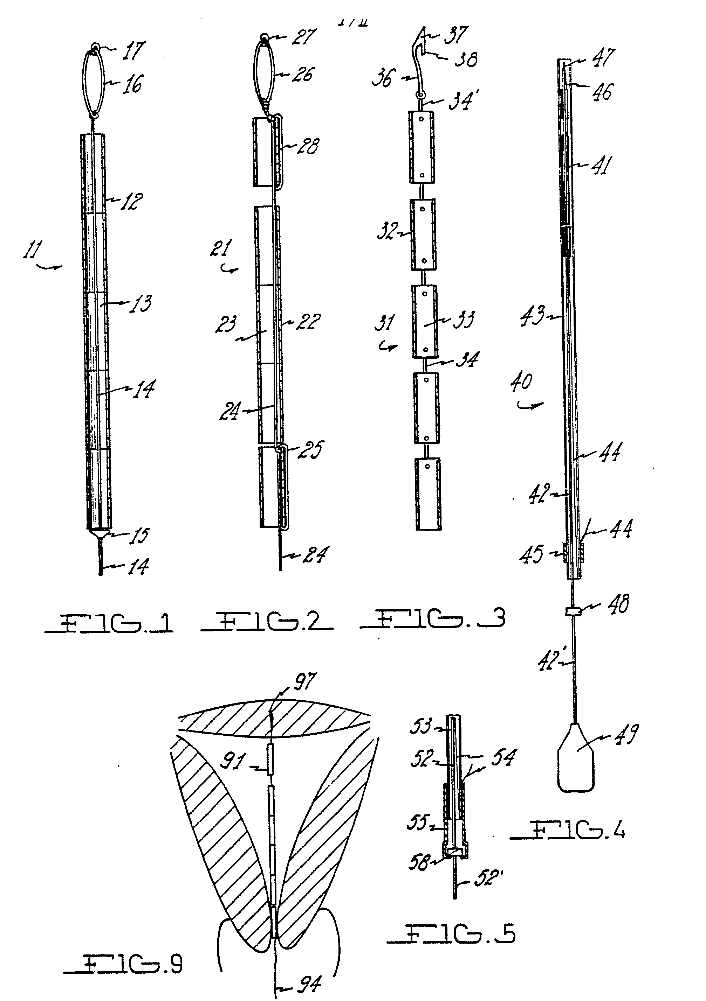

En se reportant plus particulièrement à la fig. 1, un dispositif anticonceptionnel intra-utérin 11 est constitué d'une succession d'éléments creux 12, en un matériau actif dans la cavité de la matrice, percés de part en part et disposés bout à bout pour former un conduit longitudinal 13, et d'un fil 14, traversant le dit conduit longitudinal 13. Le fil 14 est solidaire, à une extrémité du conduit 13, d'un dispositif d'arrêt 15 et est pourvu, au-delà de l'autre extrémité de ce conduit, d'une boucle 16 permettant la coopération avec une aiguille fendue. Dans la boucle 16 est formé un noeud 17 constituant dispositif d'accrochage au tissu de la matrice.

Le dispositif d'arrêt 15 assure la retenue des éléments creux, et ainsi la solidarisation du dispositif anticonceptionnel intra-utérin une fois le dispositif d'accrochage 17 inséré dans le tissu de la matrice. La distance entre le dispositif d'accrochage 17 et le dispositif d'arrêt 15 est choisie pour que la retenue des éléments 12 soit assurée de manière lâche, et qu'ainsi le dispositif anticonceptionnel intra-utérin ne constitue pas un ensemble rigide.

Suivant le mode de réalisation de la fig. 2, un dispositif anticonceptionnel intra-utérin 21 est constitué d'une succession d'éléments creux 22, en un matériau actif dans la cavité de la matrice, disposés tout à bout pour former un conduit longitudinal 23, et d'un fil 24, traversant le dit conduit longitudinal 23. A une extrémité du fil 24 est formée une boucle 26, permettant la coopération avec une aiguille fendue, et dans cette boucle, un noeud 27 constitue dispositif d'accrochage au tissu de la matrice. Une boucle 25 du fil 24, nouée autour de l'élément creux le plus éloigné du dispositif d'accrochage 27 au tissu de la matrice, forme dispositif d'arrêt, tandis qu'une boucle 28, nouée autour de l'élément creux le plus proche du dispositif d'accrochage 27 au tissu de la matrice forme second dispositif d'arrêt, les autres éléments creux étant maintenus entre ces deux dispositifs d'arrêt.

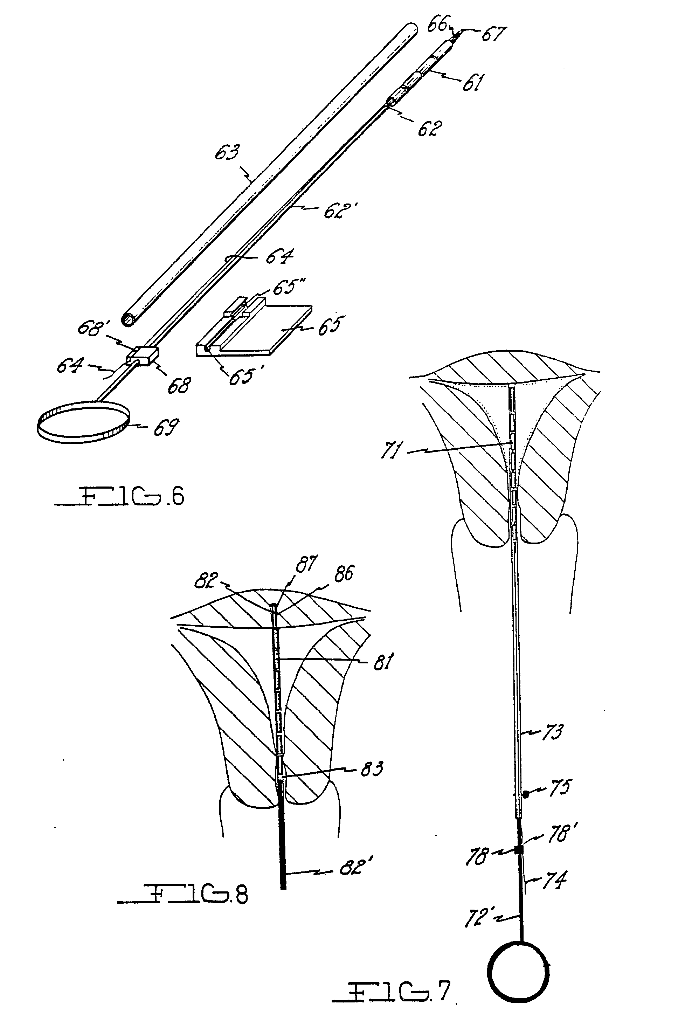

Suivant le mode de réalisation de la fig. 3, un dispositif anticonceptionnel intra-utérin 31 est constitué d'une succession d'éléments ceux 32, assemblés bout à bout et espacés entre-eux tout en étant rattachés l'un à lautre de manière à constituer un canal longitudinal 33 permettant le passage d'une aiguille, la liaison entre les différents éléments 32 étant assurée par des maillons 34. Le maillon supérieur 34' est solidaire d'un fil 36 terminé par un dispositif d'accrochage 37 au tissu de la matrice, en forme de pointe. Cette pointe comporte à sa partie inférieure un ergot 38 destiné à coopérer avec le canal d'une aiguille creuse sans tranchant, en vue de son insertion dans le tissu de la matrice.

Les divers dispositifs anticonceptionnels intra-utérins décrits ci-avant peuvent être insérés dans la matrice à l'aide d'un dispositif tel que représenté en fig. 4. Dans cette figure, le dispositif d'insertion 40 et le dispositif anticonceptionnel intra-utérin 41 sont représentés approximativement à l'échelle 1/1, du moins pour ce qui concerne les dimensions transversales, et on s'aperçoit immédiatement de l'encombrement extrêmement réduit en section transversale de l'ensemble, qui rend le franchissement du col de la matrice très aisé, même chez des femmes qui n'ont pas encore été gravides. A titre d'exemple, mentionnons qu'au cours des essais en vue de la mise au point de l'invention, il a été fait usage d'un dispositif d'insertion expérimental d'un diamètre externe d'environ 3 mm., ce qui est très largement inférieur aux dispositifs généralement utilisés, dont le diamètre se situe aux environs de 5 à 6 mm, et même au-delà.

Le dispositif d'insertion 40 est constitué d'une aiguille 42, destinée à coopérer avec le dispositif d'accrochage au tissu de la matrice formé sur le dispositif anticonceptionnel intra-utérin, et d'un élément 43 de protection de l'aiguille et de réception du dispositif anticonceptionnel intra-utérin. L'aiguille 42 passe dans le canal longitudinal du dispositif anticonceptionnel intra-utérin 41. Dans l'exemple représenté, le dispositif anticonceptionnel intra-utérin est du type représenté en fig. 2. L'aiguille 42 est fendue à son extrémité avant pour recevoir une boucle 46 portant un élément d'accrochage 47 au tissu de la matrice. L'élément 43 de protection de l'aiguille et de réception du dispositif anticonceptionnel intra-utérin est de forme tubulaire, et sa section interne correspond sensiblement à la section transversale du dispositif anticonceptionnel intra-utérin 41. Un fil 44, qui assure la solidarisation des éléments du dispositif anticonceptionnel intra-utérin, se poursuit au-delà du dispositif anticonceptionnel intra-utérin dans l'élément de protection 43, et sort à la partie inférieure de celui-ci, où il est maintenu sur la face externe du dit élément de protection 43 par une pince 45. La liaison ainsi assurée du fil à l'élément de protection empêche l'aiguille de faire saillie au-delà du dit élément de protection 43. Par ailleurs, l'aiguille 42, dont la partie arrière constitue élément d'actionnement 42', porte une butée 48 destinée à coopérer avec l'élément de protection 43 pour limiter la pénétration de l'aiguille 42 dans la matrice. Un poucier 49, formé à l'extrémité de l'aiguille, permet une manoeuvre aisée de cette dernière.

Lors de l'introduction du dispositif dans la matrice, l'aiguille 42 et le dispositif de protection 43 sont maintenus solidairement par le praticien, le fil 44 étant à l'état tendu. Le dispositif de protection est inséré dans le col de l'utérus et, vu sa faible section, il le franchit facilement. L'ensemble est alors avancé, le dispositif de protection 43 et l'aiguille 42 étant toujours maintenus solidairement, jusqu'à ce que l'extrémité avant du dispositif de protection vienne au contact de la paroi du fond de la matrice. La fig 7 illustre cette position, en liaison toutefois avec un autre dispositif d'insertion. La pince 45 est alors retirée et l'aiguille 42, solidaire de l'élément de fixation 47, est avancée dans le tissu de la matrice jusqu'à ce que la butée 48 vienne au contact de l'élément de protection 43. Une position similaire est illustrée en fig 8. L'aiguille 42 est alors retirée, abandonnant l'élément d'accrochage 47 dans le tissu de la matrice, puis le dispositif de protection 43 est ramené en-dehors de la matrice. A ce moment, l'élément d'accrochage 47, retenu dans le tissu de la matrice, assure le maintien du dispositif anticonceptionnel intra-utérin qui coulisse librement hors de l'élément de protection 43.

Dans ce mode de réalisation, la solidarisation de l'aiguille 42 et de l'élément de protection 43, à l'encontre d'un recul de l'aiguille dans l'élément de protection, est assurée par le maintien simultané de ces deux éléments dans la main du praticien. La solidarisation de l'aiguille 42 et de l'élément de protection 43, à l'encontre de l'avancement de l'aiguille, est assurée par la tension du fil 44, maintenu par la pince 45.

Dans un mode de réalisation simplifié, on pourrait prévoir que la tension du fil également soit maintenue par la prise assurée par le praticien, celui-ci maintenant simultanément l'extrémité de l'élément de protection 43, le fil 44 replié à l'extérieur de cet élément de protection, et la partie de l'aiguille 42 adjacente à l'extrémité de l'élément de protection. Dans cette perspective, la pince 45 est superflue.

Dans un autre mode de réalisation, tel que représenté partiellement en fig. 5, on peut prévoir une pince 55 qui assure le maintien du fil 54 le long de la paroi externe de l'élément de protection 53, et qui simultanément coopère avec la butée 58 pour solidariser l'aiguille 52 de l'élément de protection 53. Cette solidarisation de l'aiguille 52 à l'élément de protection 53, et de l'élément de protection 53 au fil 54 d'une part empêche l'aiguille de faire saillie de l'élément de protection, et d'autre part garantit la coopération de l'aiguille et de l'élément d'accrochage au tissu de la matrice, une tension étant maintenue dans le fil pour assurer cette coopération. Dans ce dernier cas, le praticien doit uniquement veiller à une introduction correcte du dispositif dans la matrice.

En se reportant à la fig 6, qui illustre un mode de réalisation préféré de l'invention, le dispositif d'insertion est constitué d'une aiguille 62, dont la partie arrière, constituant organe d'actionnement 62', se termine par un anneau 69, d'un élément de protection 63 et d'une pince 65. Sur l'aiguille 62 est formée une butée 68, destinée à limiter le recul de l'élément de protection 63 à la distance nécessaire pour dégager la pointe de l'aiguille sur la longueur de pénétration dans le tissu de la matrice. Cette butée 68 comporte une fente 68'. La pince 65 comprend un logement 65', destiné à coopérer avec l'aiguille 62, qui se prolonge en un logement 65'', destiné à coopérer avec l'élément de protection 63. La venue en prise de la pince 65 respectivement avec l'aiguille 62 et l'élément de protection 63, assure la solidarisation de l'aiguille à l'élément de protection.

Ce dispositif d'insertion présente de grands avantages, tant sur le plan de la fabrication que sur le plan de l'utilisation. En effet, lors de la fabrication, le dispositif anticonceptionnel intra-utérin est placé sur l'aiguille 62 en solidarisant la boucle 66 de l'extrémité de l'aiguille. Ensuite, le fil 64 est tendu et coincé dans la fente 68'. Le dispositif anticonceptionnel intra-utérin 61 est ainsi fermement solidarisé de l'aiguille, et l'on peut, sans devoir prendre de précautions particulières pour le maintien de la solidarisation du dispositif anticonceptionnel intra-utérin à l'aiguille, achever le montage du dispositif en glissant par dessus l'aiguille et le dispositif anticonceptionnel intra-utérin le dispositif de protection 63, et en ajustant sur l'ensemble la pince 65.

Lors de l'utilisation, tant l'extrémité du fil dépassant la fente 68' que la pince 65 sont facilement accessibles et permettent une manoeuvre aisée du dispositif.

Le dispositif d'insertion illustré en fig 7 est sensiblement similaire à celui de la fig 6, à la différence près que la pince 65 y est remplacée par une goupille 75 traversant l'élément de protection 73 et l'aiguille 72, à hauteur de l'élément d'actionnement 72' de cette dernière. Dans cette fig 7, le dispositif d'insertion a franchi le canal cervical et été amené au contact de la paroi du fond de la matrice, sans toutefois que l'élément de protection 73 ait été désolidarisé de l'aiguille 72, ni que le fil 74 ait été libéré de la fente 78'.

La fig 8 illustre la phase suivante du placement du dispositif anticonceptionnel intra-utérin, après libération du fil et désolidarisation de l'élément de protection 83 de l'aiguille 82. L'extrémité de l'aiguille 82, solidaire de la boucle 86 et de l'élément d'accrochage 87, est poussée dans le tissu de la matrice. De la sorte, l'élément de protection 83 recule sur l'aiguille jusqu'à venir au contact de la butée prévue sur cette dernière. La pénétration de l'aiguille dans le tissu de la matrice est ainsi limitée. Lors du retrait de l'aiguille 82, le dispositif d'accrochage 87, solidaire de la boucle 86, demeure dans le tissu de la matrice, et retient en place le dispositif anticonceptionnel intra-utérin 81 lorsque l'élément de protection 83 est à son tour retiré.

On se retrouve ainsi dans la situation illustrée en fig 9 où un dispositif anticonceptionnel intra-utérin 91, solidaire d'un fil 94, demeure maintenu dans la matrice par l'action de l'élément d'accrochage 97 situé dans le tissu de la matrice. Le fil 94 est alors recoupé à la longueur désirée.

Les dispositifs anticonceptionnels intra-utérins représentés au dessin sont tous constitués d'éléments creux de forme tubulaire. Cette forme est celle qui présente le plus de facilités sur le plan de la réalisation. Il va toutefois de soi que l'invention n'est pas limitée à cette forme tubulaire des éléments creux.

En général, ces éléments seront réalisés en cuivre. A ce propos, il convient de remarquer que la présence d'un conduit tubulaire, et le fait que les éléments soient assemblés les uns aux autres de manière relativement lâche, rend ces éléments actifs tant par leur surface interne que par leur surface externe. On obtient ainsi pour chaque élément une surface active importante. En outre, la conception du dispositif anticonceptionnel intra-utérin de l'invention permet l'utilisation d'une quantité de cuivre bien supérieure à celle des dispositifs existants. Ces deux facteurs garantissent une grande efficacité du dispositif.

Par ailleurs, lorsque les éléments sont simplement enfilés sur un fil, comme représenté aux fig. 1 et 2, rien n'interdit de constituer un ou plusieurs des éléments en une matière consommable, libérant par exemple un agent de traitement dans la matrice. La disparition d'un élément ne rompt en effet pas la chaîne, et les autres éléments demeureront en place dans la matrice après disparition de l'élément en matière consommable.

Suivant un mode de réalisation préféré de l'invention, le dispositif anticonceptionnel intra-utérin 91 est prévu de longueur suffisante pour s'étendre jusque dans le col de l'utérus comme représenté en fig. 9, ceci afin de permettre une action du cuivre à cet endroit également. Une action du cuivre au niveau du col de l'utérus est en effet importante tant sur le plan de la contraception que sur un plan prophylactique (notamment action contre les gonocoques).

L'invention a été décrite et illustrée à simple titre d'exemple nullement limitatif, et il va de soi que de nombreuses modifications peuvent être apportées à sa réalisation sans s'écarter de son esprit.

**Revendications**

1. Dispositif anticonceptionnel intra-utérin, comportant un dispositif de fixation à la paroi du fond de la matrice, solidaire d'éléments en un matériau qui est acftif dans la cavité de la matrice, ces éléments étant réunis à la file en un assemblage non rigide, caractérisé en ce que les éléments sont des éléments creux (12; 22; 32), percés de part en part et disposés bout à bout, éventuellement espacés entre eux tout en étant rattachés l'un à lautre, pour former un conduit longitudinal (13; 23; 33) permettant le passage d'une aiguille ((42; 52; 62; 72; 82), et en ce que le dispositif de fixation à la paroi du fond de la matrice est un fil (14; 24; 36; 44; 54; 64; 74; 84; 94) solidaire de l'assemblage d'éléments creux et pourvu d'un dispositif d'accrochage (17; 27; 37; 47; 67; 87; 97) au tissue de la matrice destiné à être inséré à l'aide d'une aiguille.

2. Dispositif anticonceptionnel intra-utérin suivant la revendication 1, caractérisé en ce que les éléments creux (12; 22) sont enfilés sur le fil (14; 24) pourvu d'un dispositif d'accrochage au tissu de la matrice, et sont retenus au moins par un dispositif d'arrêt (15; 25) formé sur ce fil à l'opposé du dispositif d'accrochage (17; 27) au tissu de la matrice, le fil (14; 24) réalisant un moyen d'assemblage des éléments du dispositif anticonceptionnel intra-utérin au moins lorsque le dispositif d'accrochage est inséré dans le tissu de la matrice.

3. Dispositif anticonceptionnel intra-utérin suivant la revendication 2, caractérisé en ce que le dispositif d'arrêt (25) sur le fil (24) est constitué par la solidarisation du fil à l'élément creux le plus éloigné du dispositif d'accrochage (27) au tissu de la matrice.

4. Dispositif anticonceptionnel intra-utérin suivant les revendications 1 à 3, caractérisé en ce qu'un second dispositif d'arrêt (28) est formé sur le fil (24), par solidarisation du fil à l'élément creux le plus proche du dispositif d'accrochage (27) au tissu de la matrice, la distance entre les deux dispositifs d'arrêt (25, 28) assurant un assemblage lâche des éléments creux.

5. Dispositif anticonceptionnel intra-utérin suivant l'une quelconque des revendications 2 à 4, caractérisé en ce que, en vue du traitement de la matrice, au moins certains des éléments creux (12; 22) sont réalisés en une matière consommable libérant un agent de traitement dans la matrice.

6. Dispositif anticonceptionnel intra-utérin suivant la revendication 1, caractérisé en ce que les éléments creux (32) sont assemblés l'un à l'autre par des maillons (34) réalisant un assemblage non rigide, et en ce que l'un au moins des éléments creux ou des maillons (34') est solidaire du fil (36) pourvu du moyen d'accrochage (37, 38) à la matrice.

7. Dispositif anticonceptionnel intra-utérin suivant l'une quelconque des revendications 1 à 4 et 6, caractérisé en ce que les éléments creux sont des éléments tubulaires en cuivre.

8. Dispositif anticonceptionnel intra-utérin suivant l'une quelconque des revendications précédentes, caractérisé en ce que, au-delà de son extrémité opposée au dispositif d'accrochage à la matrice (17; 27; 47; 67; 87; 97), le dispositif anticonceptionnel intra-utérin se prolonge par un fil (14; 24; 44; 54; 64; 74; 94) permettant d'assurer une traction sur le dit dispositif d'accrochage à la matrice.

9. Dispositif anticonceptionnel intra-utérin suivant la revendication 8, caractérisé en ce que le fil (14; 24; 44; 54; 64; 74; 84; 94) permettant d'assurer une traction sur le dispositif d'accrochage au tissu de la matrice est le fil portant le dit dispositif d'accrochage.

10. Dispositif d'insertion et de fixation à la matrice muni d'un dispositif anticonceptionnel intra-utérin suivant l'une quelconque des revendications 1 à 9, qui comprend une aiguille (42; 52; 62; 72; 82) pour l'introduction de l'élément d'accrochage (17; 27; 37; 47; 67; 87) solidaire du fil (14; 24; 36; 44; 54; 64; 74; 94) dans le tissu de la matrice, un élément (43; 53; 63; 73; 83) de protection de l'aiguille et de réception du dispositif anticonceptionnel intra-utérin, un élément d'actionnement (42'; 52'; 62'; 72'; 82') de l'aiguille, mobile par rapport à l'élément de protection, caractérisé en ce que l'aiguille (42; 52; 62; 72; 82) traverse le conduit (13; 23; 33) formé dans le dispositif anticonceptionnel intra-utérin pour venir en prise avec le dispositif d'accrochage (17; 27; 37; 47; 67; 87; 97) au tissu de la matrice, et en ce que la section interne de l'élément (43; 53; 63; 73; 83) de protection de l'aiguille et de réception du dispositif anticonceptionnel intra-utérin correspond substantiellement à la section transversale externe des éléments creux du dispositif anticonceptionnel intra-utérin (11; 21;31; 41; 61;71; 81; 91).

11. Dispositif d'insertion suivant la revendication 10, caractérisé en ce qu'il comprend un moyen de blocage (45; 55; 68'; 78') de l'extrémité du fil (14; 24; 44; 54; 64; 74; 84; 94) opposée à l'élément d'accrochage, et une butée (48; 58; 68; 78) limitant le recul de l'élément de protection (43; 53; 63; 73; 83) sur l'aiguille (42; 52; 62; 72; 82)

12. Dispositif d'insertion suivant la revendication 11, caractérisé en ce que le moyen de blocage du fil est formé sur l'aiguille (62; 72) et est constitué d'une fente (68'; 78') dans la butée (68; 78).

13. Dispositif d'insertion suivant l'une quelconque des revendications 10 à 12, caractérisé en ce qu'il présente des moyens de solidarisation libérables (55; 65; 75) de l'élément de protection (53; 63; 73) à l'aiguille (52; 62; 72).

**Patentansprüche**

1. Intrauterines Empfängnisverhütungsmittel, umfassend eine Vorrichtung zur Befestigung am Gebärmuttergrund und mit dieser verbundene Elemente aus einem Material, das in dem Gebärmutterhohlraum aktiv ist, wobei diese Elemente an einem Faden zu einer nicht starren Anordnung zusammengefügt sind, dadurch <u>gekennzeichnet</u>, daß diese Elemente Hohlelemente (12, 22, 32) sind mit einem von einem Ende zum anderen durchgehenden Hohlraum, die mit einander zugwandten Enden angeordnet und gegebenenfalls mit Abstand voneinander miteinander verbunden sind, um eine längliche Röhre (13, 23, 33) zu bilden, die das Hindurchführen einer Nadel (42, 52, 62, 72, 82) erlaubt, und daß die Vorrichtung zur Befestigung am Gebärmuttergrund ein Faden (14, 24, 36, 44, 54, 64, 74, 84, 94) ist, der mit der Anordnung von Hohlelementen verbunden und mit einer Vorrichtung zum Verankern im Gebärmuttergewebe versehen ist, die zum Einsetzen mit Hilfe einer Nadel bestimmt ist.

2. Intrauterines Empfängnisverhütungsmittel nach Anspruch 1, dadurch gekennzeichnet, daß die Hohlelemente (12, 22) auf dem Faden (14, 24) aufgereiht sind, der mit einer Vorrichtung zur Verankerung im Gebärmuttergewebe versehen ist, und daß sie von mindestens einer Kaltevorrichtung (15, 25) festgehalten werden, die an diesem Faden an der der Vorrichtung (17, 27) zur Verankerung im Gebärmuttergewebe entgegengesetzten Seite ausgebildet ist, wobei der Faden (14, 24) ein Verbindungsmittel für die Elemente des intrauterinen Empfängnisverhütungsmittels darstellt zumindest bis die Vorrichtung zur Verankerung in das Gebärmuttergewebe eingesetzt ist.

3. Intrauterines Empfängnisverhütungsmittel nach Anspruch 2, dadurch gekennzeichnet, daß die Haltevorrichtung (25) an dem Faden (24) von einer Verbindung des Fadens mit dem Hohlelement gebildet ist, welches am weitesten von der Vorrichtung (27) zur Verankerung im Gebärmuttergewebe entfernt ist.

4. Intrauterines Empfängnisverhütungsmittel nach den Ansprüchen 1 bis 3, dadurch gekennzeichnet, daß eine zweite Haltevorrichtung (28) an dem Faden (24) durch eine Verbindung des Fadens mit dem Hohlelement gebildet ist, das der Vorrichtung zum Verankern im Gebärmuttergewebe am nächsten ist, wobei der Abstand zwischen den beiden Haltevorrichtungen (25, 28) eine lokkere Verbindung der Hohlelemente gewährleistet.

5. Intrauterines Empfängnisverhütungsmittel nach einem der Ansprüche 2 bis 4, dadurch gekennzeichnet, daß zur Behandlung der Gebärmutter mindestens einige Hohlelemente (12, 22) aus einem abbaubaren Material bestehen, und einen Behandlungswirkstoff in der Gebärmutter freisetzen.

6. Intrauterines Empfängnisverhütungsmittel nach Anspruch 1, dadurch gekennzeichnet, daß die Hohlelemente (32) miteinander über Kettenglieder (34) verbunden sind, die eine nicht starre Verbindung bewirken, und daß mindestens eines der Hohlelemente oder der Kettenglieder (34') mit dem Faden (36) verbunden ist, der mit dem Mittel (37, 38) zur Verankerung in der Gebärmutter versehen ist.

7. Intrauterines Empfängnisverhütungsmittel nach einem der Ansprüche 1 bis 4 und 6, dadurch gekennzeichnet, daß die Hohlelemente röhrenförmige Elemente aus Kupfer sind.

8. Intrauterines Empfängnisverhütungsmittel nach einem der vorhergehenden Ansprüche, dadurch gekennzeichnet, daß das intrauterine Empfängnisverhütungsmittel über sein der Vorrichtung (17, 27, 47, 67, 97) zur Verankerung in der Gebärmutter entgegengesetztes Ende hinaus durch einen Faden (14, 24, 44, 54, 64, 74, 94) verlängert ist, der die Ausübung eines Zuges auf die Vorrichtung zur Verankerung in der Gebärmutter gestattet.

9. Intrauterines Empfängnisverhütungsmittel nach Anspruch 8, dadurch gekennzeichnet, daß der Faden (14, 24, 44, 54, 64, 74, 84, 94), der es erlaubt, einen Zug auf die Vorrichtung zur Verankerung im Gebärmuttergewebe auszuüben, jener Faden ist, der die genannte Verankerungsvorrichtung trägt.

10. Vorrichtung zum Einsetzen und Befestigen des intrauterinen Empfängnisverhütungsmittels nach einem der Ansprüche 1 bis 9 in die bzw. an der Gebärmutter, umfassend eine Nadel (42, 52, 62, 72, 82) zum Einführen des mit dem Faden (14, 24, 36, 44, 54, 64, 74, 94) verbundenen Verankerungselementes (17, 27, 37, 47, 67, 87) in das Gebärmuttergewebe, ein Element (43, 53, 63, 73, 83) zum Schutz der Nadel und zur Aufnahme des intrauterinen Empfängnisverhütungsmittels und ein Betätigungselement (42', 52', 62', 72', 82') für die Nadel, das gegenüber dem Schutzelement beweglich ist, dadurch gekennzeichnet, daß die Nadel (42, 52, 62, 72, 82) die in dem intrauterinen Empfängnisverhütungsmittel ausgebildete Röhre (13, 23, 33) durchdringt, um mit der Vorrichtung (17, 27, 37, 47, 67, 87, 97) zur Verankerung im Gebärmuttergewebe in Eingriff zu kommen, und daß der innere Querschnitt des Schutzelementes (43, 53, 63, 73, 83) für die Nadel und die Aufnahme des intrauterinen Empfängnisverhütungsmittels im wesentlichen dem äußeren Querschnitt der Hohlelemente (11, 21, 31, 41, 61, 71, 81, 91) des intrauterinen Empfängnisverhütungsmittels entspricht.

11. Einsetzvorrichtung nach Anspruch 10, dadurch gekennzeichnet, daß sie ein Arretierungsmittel (45, 55, 68', 78') zum Arretieren des dem Verankerungselement entgegengesetzten Endes des Fadens (14, 24, 44, 54, 64, 74, 84, 94) und einen Anschlag (48, 58, 68, 78) umfaßt, der das Zurückziehen des Schutzelementes (43, 53, 63, 73, 83) auf der Nadel (42, 52, 62, 72, 82) begrenzt.

12. Einsetzvorrichtung nach Anspruch 11, dadurch gekennzeichnet, daß das Arretierungsmittel zum Arretieren des Fadens an der Nadel (62, 72) ausgebildet ist und aus einem Schlitz (68', 78') im Anschlag (68, 78) besteht.

13. Einsetzvorrichtung nach einem der Ansprüche 10 bis 12, dadurch gekennzeichnet, daß sie Mittel (55, 65, 75) zur lösbaren Verbindung des Schutzelementes (53, 63, 73) mit der Nadel (52, 62, 72) aufweist.

## Claims

1. An intra-uterine contraceptive device comprising a means for attaching it to the wall of the back of the uterus, fixed with respect to elements of a material which is active in the uterine cavity, said elements being connected in a line to provide a non- rigid assembly, characterised in that the elements are hollow elements (12; 22; 32) which are apertured right through and which are disposed in end-to-end relationship, possibly spaced from each other while being connected to each other, to form a longitudinal conduit (13; 23; 33) permittihg the passage of a needle (42; 52; 62; 72; 82) and that the means for attachment to the wall of the back of the uterus is a wire or thread (14; 24; 36; 44; 54; 64; 74; 84; 94) which is fixed with respect to the assembly of hollow elements and provided with a device (17; 27; 37; 47; 67; 87; 97) for hooking to the tissue of the uterus, intended to be inserted by means of a needle.

2. An intra-uterine contraceptive device according to claim 1 characterised in that the hollow elements (12; 22) are threaded on to the wire or thread (14; 24) which is provided with a means for hooking to the tissue of the uterus, and are retained at least by a stop means (15; 25) foreed on the wire or thread opposite to the means (17; 27) for hooking to the tissue of the uterus, the wire or thread (14; 24) providing a means for assembly of the elements of the intra-uterine contraceptive device at least when the hooking means is inserted into the tissue of the uterus.

3. An intra-uterine contraceptive device according to claim 2 characterised in that the stop means (25) on the wire or thread (24) is formed by the wire or thread being fixed with respect to the hollow element which is most remote from the means (27) for hooking to the tissue of the uterus.

4. An intra-uterine contraceptive device according to claims 1 to 3 characterised in that a second stop means (28) is formed on the wire or thread (24) by fixing the wire or thread with respect to the hollow element which is closest to the means (27) for hooking to the tissue of the uterus, the distance beteeen the two stop means (25, 28) providing for loose assembly of the hollow elements.

5. An intra-uterine contraceptive device according to any one of claims 2 to 4 characterised in that, for treatment of the uterus, at least some of the hollow elements (12; 22) are made of a consumable material which liberates a treatment agent in the uterus.

6. An intra-uterine contraceptive device according to claim 1 characterised in that the hollow elements (32) are assembled to each other by links (34) providing a non-rigid assembly and that one at least of the hollow elements or the links (34') is fixed with respect to the wire or thread (36) provided with the means (37, 38) for attachment to the uterus.

7. An intra-uterine contraceptive device according to any one of claims 1 to 4 and 6 characterised in that the hollow elements are tubular copper elements.

8. An intra-uterine contraceptive device according to any one of the preceding claim characterised in that beyond its end opposite to the means (17; 27; 47; 67; 87; 97) for hooking to the uterus the intra-uterine contraceptive device is tended by a wire or thread (14; 24; 44; 54; 64; 74; 94) which makes it possible to apply a pulling force to said means for hooking to the uterus.

9. An intra-uterine contraceptive device according to claim 8 characterised in that the wire or thread (14; 24; 44; 54; 64; 74; 84; 94) which makes it possible to apply a pulling force to the means for hooking to the tissue of the uterus is the wire or thread carrying said hooking means.

10. A device for insertion and attachment to the uterus, provided with an intra-uterine contraceptive device according to any one of claims 1 to 9, which comprises a needle (42; 52; 62; 72; 82) for introducing a hooking element (17; 27; 37; 47; 67; 87) which is fixed with respect to the wire or thread (14; 24; 36; 44; 54; 64; 74; 94) into the tissue of the uterus, an element (43; 53; 63; 73; 83) for protecting the needle and receiving the intra-uterine contraceptive device, an element (42'; 52'; 62'; 72'; 82') for actuating the needle, which is movable with respect to the protection element, characterised in that the needle (42; 52; 62; 72; 82) passes through the conduit (13; 23; 33) formed in the intra-uterine contraceptive device to come into engagement with the means (17; 27; 37; 47; 67; 87; 97) for hooking to the tissue of the uterus, and that the internal section of the element (43; 53; 63; 73; 83) for protecting the needle and receiving the intra-uterine contraceptive device substantially corresponds to the external cross-section of the hollow elements of the intra-uterine contraceptive device (11; 21; 31; 41; 61; 71; 81; 91).

11. An insertion device according to claim 10 characterised in that it comprises a means (45; 55; 68'; 78') for blocking the end of the wire or thread (14; 24; 44; 54; 64; 74; 84; 94) opposite to the hooking element, and a stop (48; 58; 68; 78) for limiting the rearward movement of the protection element (43; 53; 63; 73; 83) on the needle (42; 52; 62; 72; 82).

12. An insertion device according to claim 11 characterised in that the means for blocking of the wire or thread is formed on the needle (62; 72) and is formed by a slot (68'; 78') in the stop (68; 78).

13. An insertion device according to any one of claims 10 to 12 characterised in that it has releasable means (55; 65; 75) for fixing the protection element (53; 63; 73) to the needle (52; 62; 72).

0 191 747

FIG.1   FIG.2   FIG.3   FIG.4   FIG.5   FIG.9

66
67
61
62
63
62'
64
65"
65
68'
68
64
65'
69

_F1G_.6

82
87
86
81
83
82'

_F1G_.8

71
73
75
78'
78
74
72'

_F1G_.7